# EUROPEAN PATENT APPLICATION

(11) **EP 3 150 700 A1**
(43) Date of publication of application: **05.04.2017**
(21) Application number: 15800288.1
(22) Date of filing: 28.05.2015
(51) Int. Cl.: C12N 5/071, C12N 1/00, C12N 5/10

(54) **CULTURE MEDIUM AND CULTURING METHOD FOR ANCHORAGE-DEPENDENT CELLS, CELL COMPOSITION INCLUDING STEM CELLS AND/OR DIFFERENTIATED CELLS DERIVED FROM STEM CELLS, AND PRODUCTION METHOD FOR CELL COMPOSITION**

(30) Priority: 28.05.2014 JP 2014110450
(71) Applicant: Kyowa Hakko Kirin Co., Ltd., Tokyo 100-8185 (JP)
(72) Inventor: NAKASHIMA, Yoshiki, Suita-shi Osaka 565-0871 (JP); OMASA, Takeshi, Suita-shi Osaka 565-0871 (JP); TSUKAHARA, Masayoshi, Tokyo 100-8185 (JP)
(74) Representative: Hoffmann Eitle
(86) International application number: PCT/JP2015/002694
(87) International publication number: WO 2015/182140

(57) **Abstract**

As a technique capable of culturing anchorage-dependent cells without using an anchorage, provided is a medium for anchorage-dependent cells, which comprises MFG-E8 (Milk fat globule-EGF factor 8) or a fragment of the protein. This medium can promote adhesion of anchorage-dependent cells in the absence of an anchorage, enables the survival and proliferation (colony formation) of the cells, and further, also enables the subsequent differentiation if the anchorage-dependent cells are stem cells.

SEQUENCE LISTING

<110>  KYOWA HAKKO KIRIN CO., LTD.

<120>  MEDIUM AND METHOD FOR CULTURING ANCHORAGE-DEPENDENT CELL, AND
       CELL COMPOSITION COMPRISING STEM CELL AND/OR  DIFFERENTIATING
       CELL INDUCED FROM STEM CELL AND METHOD FOR PRODUCING THEREOF

<130>  PKH-9001WO

<150>  JP2014-110450
<151>  2014-05-28

<160>  1

<170>  PatentIn version 3.5

<210>  1
<211>  387
<212>  PRT
<213>  Homo sapiens

<400>  1

Met Pro Arg Pro Arg Leu Leu Ala Ala Leu Cys Gly Ala Leu Leu Cys
1               5                   10                  15

Ala Pro Ser Leu Leu Val Ala Leu Asp Ile Cys Ser Lys Asn Pro Cys
            20                  25                  30

His Asn Gly Gly Leu Cys Glu Glu Ile Ser Gln Glu Val Arg Gly Asp
        35                  40                  45

Val Phe Pro Ser Tyr Thr Cys Thr Cys Leu Lys Gly Tyr Ala Gly Asn
    50                  55                  60

His Cys Glu Thr Lys Cys Val Glu Pro Leu Gly Met Glu Asn Gly Asn
65                  70                  75                  80

Ile Ala Asn Ser Gln Ile Ala Ala Ser Ser Val Arg Val Thr Phe Leu
                85                  90                  95

Gly Leu Gln His Trp Val Pro Glu Leu Ala Arg Leu Asn Arg Ala Gly
            100                 105                 110

Met Val Asn Ala Trp Thr Pro Ser Ser Asn Asp Asp Asn Pro Trp Ile
        115                 120                 125

Gln Val Asn Leu Leu Arg Arg Met Trp Val Thr Gly Val Val Thr Gln
    130                 135                 140

Gly Ala Ser Arg Leu Ala Ser His Glu Tyr Leu Lys Ala Phe Lys Val
145                 150                 155                 160

Ala Tyr Ser Leu Asn Gly His Glu Phe Asp Phe Ile His Asp Val Asn
                165                 170                 175

Lys Lys His Lys Glu Phe Val Gly Asn Trp Asn Lys Asn Ala Val His
            180                 185                 190

Val Asn Leu Phe Glu Thr Pro Val Glu Ala Gln Tyr Val Arg Leu Tyr
        195                 200                 205

Pro Thr Ser Cys His Thr Ala Cys Thr Leu Arg Phe Glu Leu Leu Gly
    210                 215                 220

Cys Glu Leu Asn Gly Cys Ala Asn Pro Leu Gly Leu Lys Asn Asn Ser
225                 230                 235                 240

Ile Pro Asp Lys Gln Ile Thr Ala Ser Ser Ser Tyr Lys Thr Trp Gly
                245                 250                 255

Leu His Leu Phe Ser Trp Asn Pro Ser Tyr Ala Arg Leu Asp Lys Gln
            260                 265                 270

Gly Asn Phe Asn Ala Trp Val Ala Gly Ser Tyr Gly Asn Asp Gln Trp
        275                 280                 285

Leu Gln Val Asp Leu Gly Ser Ser Lys Glu Val Thr Gly Ile Ile Thr
    290                 295                 300

Gln Gly Ala Arg Asn Phe Gly Ser Val Gln Phe Val Ala Ser Tyr Lys
305                 310                 315                 320

Val Ala Tyr Ser Asn Asp Ser Ala Asn Trp Thr Glu Tyr Gln Asp Pro
                325                 330                 335

Arg Thr Gly Ser Ser Lys Ile Phe Pro Gly Asn Trp Asp Asn His Ser
            340                 345                 350

His Lys Lys Asn Leu Phe Glu Thr Pro Ile Leu Ala Arg Tyr Val Arg
        355                 360                 365

Ile Leu Pro Val Ala Trp His Asn Arg Ile Ala Leu Arg Leu Glu Leu
    370                 375                 380

Leu Gly Cys
385

## Description

### Technical Field

The present invention relates to a medium for anchorage-dependent cells, a method for culturing anchorage-dependent cells, a cell composition comprising stem cells and/or differentiated cells derived from the stem cells, and a method for producing the cell composition. More specifically, the present invention relates to a medium for culturing anchorage-dependent cells without using an anchorage, a method for producing stem cells and the like using the aforementioned medium, etc.

### Background Art

For proliferation of anchorage-dependent cells such as CHO cells, the cells need to adhere to the surface of a base material of an incubator. In order to enable adhesion of the cells to the surface of the base material, the surface of the base material has been coated with an adhesion molecule called an anchorage, such as laminin, collagen, or polylysine. The same applies also to the culture of stem cells such as ES cells (embryonic stem cell) or iPS cells (induced pluripotent stem cell), and it has been known that proliferation of the cells is difficult in the absence of an anchorage or feeder cells. In particular, a problem in the case of human iPS cells is that differentiation cannot be induced if adhesion is insufficient.

Conventionally used anchorages include matrigel and a recombinant protein (Non Patent Literature 1). However, these materials are expensive, and are lack of stability, such that there are large differences among lots, in terms of quality. Anchorages, in which macromolecules such as polymers are used, have been developed (Non Patent Literatures 2 and 3), but these anchorages are also extremely expensive, and there may also be a case where these anchorages are unsuitable for some types of cell lines.

Milk fat globule-EGF factor 8 (MFG-E8) is a lactadherin homolog in human, and it is a membrane-associated glycoprotein found in breast milk and breast epithelial cells (see Non Patent Literature 1). Non Patent Literature 4 describes that administration of MFG-E8 can be effective for the treatment of sepsis or ischemia-reperfusion injury. Moreover, Non Patent Literature 5 describes that MFG-E8 secreted from activated macrophages binds to apoptotic cells and induces phagocytosis by phagocytes. To date, addition of MFG-E8 to a medium for animal cells, or the culture of anchorage-dependent cells or iPS cells in the presence of MFG-E8, has not yet been known.

### Citation List

### Non Patent Literature

Non Patent Literature 1: Nature Biotechnology, 2010, 28 (6) : 611-615
Non Patent Literature 2: Nature Biotechnology, 2010, 28 (6) : 606-610
Non Patent Literature 3: Nature Biotechnology, 2010, 28(6): 581-583
Non Patent Literature 4: Mol. Med., 2011, 17 (I-2) : 126-33 Non Patent Literature 5: Nature, 2002, 417(6885): 182-7

### Summary of Invention

### Technical Problem

It is a main object of the present invention to provide a technique capable of culturing anchorage-dependent cells without using an anchorage.

### Solution to Problem

The present inventors have found that MFG-E8 promotes adhesion of anchorage-dependent cells in the absence of an anchorage and enables the survival and proliferation (colony formation) of the cells, and further, the subsequent differentiation, if the anchorage-dependent cells are stem cells.

Specifically, in order to achieve the aforementioned object, the present invention provides the following [1] to [25] :
[1] A medium for an anchorage-dependent cell, which comprises MFG-E8 (Milk fat globule-EGF factor 8) or a fragment of the protein.
[2] The medium according to [1], wherein the anchorage-dependent cell is a stem cell.
[3] The medium according to [2], wherein the stem cell is an embryonic stem cell, an induced pluripotent stem cell, or a mesenchymal stem cell.
[4] The medium according to any of [1] to [3], wherein the anchorage-dependent cell is derived from a human.
[5] The medium according to any of [1] to [4], which is a serum-free medium.
[6] An adhesion promoter for an anchorage-dependent cell, which comprises MFG-E8 or a fragment of the protein.
[7] A method for culturing an anchorage-dependent cell, which comprises a step of culturing an anchorage-dependent cell in a medium comprising MFG-E8 or a fragment of the protein in the absence of an anchorage.
[8] The culture method according to [7], wherein the anchorage-dependent cell is a stem cell.
[9] The culture method according to [8], wherein the stem cell is an embryonic stem cell, an induced pluripotent stem cell, or a mesenchymal stem cell.
[10] The culture method according to any of [7] to [9], wherein the anchorage-dependent cell is derived from a human.
[11] The culture method according to any of [7] to [10], wherein the medium is a serum-free medium.
[12] A method for producing a cell composition comprising a stem cell, wherein the method comprises a step of culturing a stem cell in a medium comprising MFG-E8 or a fragment of the protein in the absence of an anchorage.
[13] A method for producing a cell composition comprising a stem cell, wherein the method comprises:
   (a) a step of culturing a stem cell on a feeder cell,
   (b) a step of dissociating the stem cell from the feeder cell, and
   (c) a step of culturing the stem cell in a medium comprising MFG-E8 or a fragment of the protein in the absence of a feeder cell.
[14] The method according to [13], wherein the medium used in the step (c) is a serum-free medium.
[15] The method according to any of [12] to [14], wherein the stem cell is an embryonic stem cell, an induced pluripotent stem cell, or a mesenchymal stem cell.
[16] The method according to any of [12] to [15], wherein the stem cell is derived from a human.
[17] A method for producing a cell composition comprising a differentiated cell from a stem cell, wherein the method comprises a step of inducing differentiation of a stem cell in a medium comprising MFG-E8 or a fragment of the protein in the absence of an anchorage.
[18] The method according to [17], wherein the stem cell is an embryonic stem cell, an induced pluripotent stem cell, or a mesenchymal stem cell.
[19] The method according to [17] or [18], wherein the stem cell is derived from a human.
[20] The method according to any of [17] to [19], wherein the medium is a serum-free medium.
[21] A cell composition comprising a stem cell and/or a differentiated cell derived from the stem cell, cultured in a medium comprising MFG-E8 or a fragment of the protein in the absence of an anchorage.
[22] The cell composition according to [21], wherein the stem cell is an embryonic stem cell, an induced pluripotent stem cell, or a mesenchymal stem cell.
[23] The cell composition according to [21] or [22], wherein the stem cell is derived from a human.
[24] The cell composition according to any of [21] to [23], wherein the medium is a serum-free medium.
[25] The cell composition according to any of [21] to [24], which comprises MFG-E8 or a fragment of the protein.

### Advantageous Effects of Invention

According to the present invention, a technique capable of culturing anchorage-dependent cells without using an anchorage is provided.

### Brief Description of Drawings

[Figure 1] Figure 1 is a view showing the results obtained by evaluating the effects of MFG-E8 in the case of culturing anchorage-dependent cells in the absence of an anchorage, using human iPS cells (Example 1). The human iPS cells were cultured on a plate that had not been coated with an anchorage in the presence or absence of MFG-E8 for 4 days, and the area of growing colonies was measured.
[Figure 2] Figure 2 is a view showing the results obtained by evaluating the effects of MFG-E8, while changing the concentration of MFG-E8 added (Example 1).

### Description of Embodiments

Hereinafter, a preferred embodiment for carrying out the present invention will be described. It is to be noted that the embodiment described in the following shows an example of the representative embodiments of the present invention, and thus that the scope of the present invention should not be narrowly construed by the following embodiment.

### 1. Medium for anchorage-dependent cells

### [MFG-E8]

The medium for anchorage-dependent cells according to the present invention is characterized in that it comprises MFG-E8 (Milk fat globule-EGF factor 8) or a fragment of the protein.

The amino acid sequence of human MFG-E8 is shown in SEQ ID NO: 1 (GenBank Accession No. Q08431-1; http://www.uniprot.org/uniprot/Q08431). The amino acid sequence of human MFG-E8 may also be GenBank Accession No. Q08431-2 or No. Q08431-3 (http://www.uniprot.org/uniprot/Q08431). The amino acid sequence consisting of amino acids at positions 1 to 23 in SEQ ID NO: 1 is a secretory signal sequence. MFG-E8 is also referred to as lactadherin, medin, or secreted EGF repeat and discoidin domains-containing protein 1 (SED1).

The MFG-E8 used in the medium according to the present invention is not limited to human MFG-E8 shown in SEQ ID NO: 1, and it may be MFG-E8 other than human MFG-E8 (MFG-E8 homolog), as long as it has an activity of promoting adhesion of anchorage-dependent cells. Such MFG-E8 homologs may be derived from any animals, and thus, they may be homologs, for example, from rodents such as a rat, a mouse, a hamster, or a guinea pig, Lagomorpha such as a rabbit, ungulates such as a swine, a bovine, a goat, or sheep, Carnivora such as a dog or a cat, primates such as a human, a monkey, a rhesus monkey, a marmoset, an orangutan, or a chimpanzee. As an example, mouse-derived MFG-E8 is described in PLoS ONE, 2012, 7, e36368 and PLoS ONE, 2011, 6, e27685. The MFG-E8 homolog used in the medium according to the present invention can be selected, as appropriate, depending on the origin of anchorage-dependent cells to be cultured, and preferably, an MFG-E8 homolog derived from the same animal as that as an origin of anchorage-dependent cells to be cultured is used. For example, in a case where human-derived cells are cultured, human-derived MFG-E8 is preferably used.

In addition, the MFG-E8 used in the medium according to the present invention comprises a polypeptide having any given length (a fragment of MFG-E8) that can be generated from the amino acid sequence of human MFG-E8 or an MFG-E8 homolog, as long as it has an activity of promoting adhesion of anchorage-dependent cells. The length of the polypeptide is, for example, 21 to 100, preferably 101 to 200, and more preferably 201 to 364 amino acid residues. Specific examples of a preferred fragment include a polypeptide consisting of an amino acid sequence of amino acids at positions 24 to 387 in SEQ ID NO: 1 (364 amino acid residues). It is to be noted that when the "polypeptide" is used in the present description, it is used exchangeably with a "peptide" or a "protein." Hereinafter, the "MFG-E8 and a fragment thereof" may be simply referred to as "MFG-E8" at times.

Moreover, the MFG-E8 used in the medium according to the present invention may also be a mutant protein consisting of an amino acid sequence formed by a deletion, substitution, insertion, or addition of one or several amino acids in the amino acid sequence of human MFG-E8 and an MFG-E8 homolog, as long as it has an activity of promoting adhesion of anchorage-dependent cells.

Examples of the amino acid sequence of such a mutant protein include an amino acid sequence formed by adding a His tag sequence to an amino acid sequence formed by a deletion of a signal sequence in the amino acid sequence shown in SEQ ID NO: 1 (an amino acid sequence consisting of amino acids at positions 24 to 387).

The "amino acid sequence formed by a deletion, substitution, insertion, or addition of one or several amino acids" means an amino acid sequence formed by a deletion, substitution, insertion, or addition of almost as many amino acids as those that can be deleted, substituted, inserted, or added according to a known mutant polypeptide production method such as site-directed mutagenesis (preferably 10 or less, more preferably 7 or less, and further preferably 5 or less amino acids). It has been well known in the present technical field that several amino acids in the amino acid sequence of a protein can be easily modified without significantly influencing the structure or function of the protein.

Furthermore, it has also been well known that a native protein includes mutants that do not significantly change the structure or function of the native protein. Accordingly, the "amino acid sequence formed by a deletion, substitution, insertion, or addition of one or several amino acids" is not limited to a sequence into which a mutation has been artificially introduced according to a known mutant polypeptide production method, and it can also be the amino acid sequence of a mutant existing in the nature.

The MFG-E8 used in the medium according to the present invention may be a protein isolated and/or purified from the nature, a recombinant protein, or a synthetic protein, or may also be a chemically modified protein. Isolation of a native protein, the expression of a recombinant protein, the synthesis of a protein, or purification of these proteins can be carried out according to conventionally known methods.

### [Basal medium]

The composition of the medium according to the present invention may be the same as that of a medium conventionally used for the culture of anchorage-dependent cells, with the exception that the present medium comprises MFG-E8. Examples of such a medium include a medium used for the culture of cells derived from animal tissues. Specific examples include the following media.

RPMI-1640 medium, Eagle's MEM medium, Dulbecco's modified MEM medium, GMEM (Glasgow's MEM), α-MEM, 199 medium, IMDM medium, DMEM medium, Hybridoma Serum free medium (Invitrogen), Chemically Defined Hybridoma Serum Free medium (Invitrogen), Ham's Medium F-12, Ham's Medium F-10, Ham's Medium F12K, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's MB752/1, CMRL-1066, Williams' medium E, Brinster's BMOC-3 Medium, Essential 8 Medium (Life Technologies), mTeSR1 (STEMCELL Technologies), TeSR-E8 medium (STEMCELL Technologies), StemSure (Wako Pure Chemical Industries, Ltd.), mESF medium (Wako Pure Chemical Industries, Ltd.), StemFit (Ajinomoto Co., Inc.), S-medium (DS Pharma), ReproXF (ReproCELL), PSGro-free Human iPSC/ESC Growth Medium (StemRD), hPSC Growth Medium (Takara Bio Inc.), ReproFF2 (ReproCELL), EX-CELL 302 medium (SAFC), EX-CELL-CD-CHO (SAFC) or STEMdiff APEL Medium (STEMCELL Technologies), and the mixtures thereof.

The medium according to the present invention can be prepared by previously adding MFG-E8 to such a medium or by adding MFG-E8 to the medium during cell culture. The concentration of MFG-E8 added to the medium is not particularly limited, as long as it has an activity of promoting adhesion of anchorage-dependent cells. The concentration can be set, for example, from 1 µg/ml to 5 µg/ml.

In addition, physiologically active substances, nutritional factors and the like, which are necessary for the survival or proliferation of cells, can be added to the medium, as necessary. These additives may previously have been added to the medium or may be added thereto during cell culture.

Examples of the physiologically active substance include insulin, IGF-1, transferrin, albumin, and coenzyme Q₁₀.

Examples of the nutritional factor include sugar, amino acid, vitamin, a hydrolysate, and a lipid.

Examples of the sugar include: neutral sugars such as glucose, mannose, or fructose; acidic sugar such as sialic acid; amino sugar; and sugar alcohol. These sugars are used alone or in combinations of two or more.

Examples of the amino acid include L-alanine, L arginine, L-asparagine, L-aspartic acid, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, and L-valine. These amino acids are used alone or in combinations of two or more.

Examples of the vitamin include d-biotin, D-pantothenic acid, choline, folic acid, myo-inositol, niacinamide, pyridoxal, riboflavin, thiamine, cyanocobalamin, and DL-α-tocopherol. These vitamins are used alone or in combinations of two or more.

Examples of the hydrolysate include products obtained by hydrolyzing soybean, wheat, rice, green pea, corn, cottonseed, enzyme extract, etc.

Examples of the lipid include cholesterol, linoleic acid, and linolenic acid.

Moreover, antibiotics such as kanamycin, streptomycin, penicillin, or hygromycin may be added to the medium, as necessary. When an acidic substance such as sialic acid is added to the medium, it is desirable that the pH of the medium is adjusted to pH 5 to 9, and preferably pH 6 to 8, which is a neutral range suitable for the growth of cells.

According to the medium of the present invention, anchorage-dependent cells can be adhered to a base material of an incubator, without addition of serum. Therefore, the medium according to the present invention may be a medium that does not contain serum, namely, a serum-free medium. The medium according to the present invention may comprise serum, but from the viewpoint of prevention of the mixing of components derived from different animal species into the medium, the present medium preferably does not contain serum. Herein, the serum-free medium means a medium that does not contain unadjusted or unpurified serum. The serum-free medium may contain purified blood-derived components or animal tissue-derived components (e.g., a growth factor).

As with the serum, the medium according to the present invention may contain or may not contain a serum replacement. Examples of the serum replacement include albumin alternatives such as albumin, lipid-rich albumin, and recombinant albumin, vegetable starch, dextran, a protein hydrolysate, transferrin or other iron transporters, fatty acid, insulin, a collagen precursor, trace elements, 2-mercaptoethanol, 3'-thioglycerol, and the equivalents thereof. Specific examples of the serum replacement include a product prepared by the method described in International Publication No. WO 98/30679, commercially available Knockout Serum Replacement (KSR), Chemically-defined Lipid concentrated (Life Technologies), and Glutamax (Life Technologies).

### [Anchorage-dependent cells]

In the present invention, the "anchorage-dependent cells" comprise a broad range of cells, which need to adhere to something for their survival and/or proliferation, and the survival and/or proliferation of which is impossible or is significantly inhibited, if they are in a non-adhered state or a suspending state. The "anchorage-dependent cells" are not particularly limited, as long as they are animal tissue-derived cells, which can adhere to a base material of an incubator in the presence of MFG-E8, without any anchorages, and can proliferate thereon.

Herein, the "anchorage" broadly includes a protein constituting an extracellular matrix in a living body, a recombinant protein thereof, a synthetic protein thereof, modified products of these proteins, partial polypeptides, cell-adhesion synthetic molecules, and the like. Specific examples of the anchorage include collagen, gelatin, poly-L-lysine, poly-D-lysine, laminin, a partial structure of laminin, fibronectin, the mixtures thereof (e.g., matrigel), and a cell membrane lysate preparation (Lancet, 2005, 365, pp. 1636-1641). Conventionally known anchorage materials are listed below: organic materials selected from the biopolymer group consisting of collagen, gelatin, hyaluronic acid, proteoglycan, chitin, chitosan, a chitosan derivative, fibrin, dextran, agarose, calcium alginate, silk, and a combination thereof; and synthetic polymer materials selected from the group consisting of aliphatic polyester, poly(amino acid), poly(propylene fumarate), copoly(ether-ester), polyorthoester, polyalkylene oxalate, polyamide, polycarbonate, polycaprolactone, poly(iminocarbonate), polyorthoester, polyoxaester, polyamide ester, polyoxaester comprising an amine group, polyanhydride, polyphosphazene, polyurethane, hydroxybutyrate, dioxanone, hydrogel such as polyacrylate, polyvinyl alcohol, polyethylene glycol, or polyethyleneimine, and any given copolymers thereof, mixtures thereof, and chemical derivatives thereof. The aliphatic polyester may be polylactic acid, polyglycolic acid, a copolymer thereof, or a mixture thereof.

The species from which anchorage-dependent cells are derived, to which the medium according to the present invention can be applied, is not particularly limited and such cells may be cells of, for example, rodents such as a rat, a mouse, a hamster, or a guinea pig, Lagomorpha such as a rabbit, ungulates such as a swine, a bovine, a goat, or sheep, Carnivora such as a dog or a cat, and primates such as a human, a monkey, a rhesus monkey, a marmoset, an orangutan, or a chimpanzee.

Specific examples of the anchorage-dependent cells, to which the medium according to the present invention can be applied include: Chinese hamster ovary-derived CHO cells, CHO-K1 (ATCC CCL-61), CHO/dhfr- (ATCC CRL-9096), Pro5 cell line (ATCC CRL-1781), CHO-S (manufactured by Invitrogen; Cat#11619); baby hamster kidney-derived BHK cells; human cervical cancer-derived HeLa cells; mouse breast cancer-derived C-127 cells; mouse fibroblasts NIH/3T3 and BALB3T3; African green monkey kidney-derived VerotsS3; mouse cell lines NS0 (ATCC CRL-1827) and SP2/0 (ATCC CRL-1581); mouse myeloma cell line SP2/0-Ag14; rat myeloma cell lines Y3 Ag1.2.3. (ATCC CRL-1631), YO (ECACC No: 85110501), YB2/3HL.P2.G11.16Ag.20, and YB2/0 (ATCC CRL-1662); Syrian hamster kidney tissue-derived cells BHK-21 (ATCC CCL-10) and MDCK (ATCC CCL-34); human cell lines HEK293 and PER.C6 (ECACC 96022940); human leukemia cell lines Namalwa cells or NM-F9 cells; hybridoma cells; fertilized egg cells; and stem cells.

Specific examples of the anchorage-dependent cells that are stem cells include: mesenchymal stem cells that differentiate into myoblasts, vascular endothelial cells, osteoblasts, adipocytes, muscle cells, cardiomyocytes, chondrocytes, etc.; neural stem cells that differentiate into neurons or glial cells; hematopoietic stem cells or bone marrow stem cells that differentiate into leukocytes, erythrocytes, platelets, mast cells, dendritic cells, etc.; and pluripotent stem cells, such as embryonic stem cells (ES cells) or induced pluripotent stem cells (iPS cells), which have been known to proceed to a step of differentiation and/or induction into various tissues through formation of a pseudo-embryo called embryoid body (EB body) from the state of a spheroid, embryonic germ (EG) cells derived from primordial germ cells, multipotent germline stem (mGS) cells isolated in the process of the establishment of GS cells from testicular tissues and the culture thereof, and multipotent adult progenitor cells (MAPC) isolated from bone marrow.

More specific examples of the anchorage-dependent cells that are stem cells include: a C2C12 cell line known to differentiate into a myotube; a 3T3-L1 cell line known to differentiate into adipocytes; mouse neural stem cells (MNSC) derived from the cerebral striatum of a mouse fetus; rat neural stem cells (RNSC) derived from the midbrain of a rat fetus; and the cell line 3'-mRLh-2 derived from rat liver.

Herein, the "stem cells" as subjects of the present invention are not limited to those described above and mean immature cells having self-replication ability and differentiation proliferation ability. Depending on differentiation ability, such stem cells include pluripotent stem cells, multipotent stem cells, and unipotent stem cells. The "stem cells" are generally defined as undifferentiated cells having both "self-reproduction ability" by which the cells can proliferate, while maintaining their undifferentiated state, and "differentiation pluripotency" by which the cells can differentiate into all of tridermic series.

The pluripotent stem cells mean cells having an ability to differentiate into all of tissues or cells that constitute a living body.

The multipotent stem cells mean cells having an ability to differentiate into not all types of, but a plurality of types of, tissues or cells.

The unipotent stem cells mean cells having an ability to differentiate into specific tissues or cells.

Examples of the pluripotent stem cells particularly include the aforementioned ES cells and iPS cells. Stem cells, which have been established by culturing an early embryo produced by transplantation of the nucleus of a somatic cell, are also preferable as pluripotent stem cells (Nature, 1997, 385, 810; Science, 1998, 280, 1256; Nature Biotechnology, 1999, 17, 456; Nature, 1998, 394, 369; Nature Genetics, 1999, 22, 127; Proc. Natl. Acad. Sci. USA, 1999, 96, 14984; Nature Genetics, 2000, 24, 109).

Human ES cell lines, for example, WA01 (H1) and WA09 (H9) are available from WiCell Research Institute, and KhES-1, KhES-2, and KhES-3 are available from Institute for Frontier Medical Sciences, Kyoto University (Kyoto, Japan).

Examples of the iPS cells include cells having the same pluripotency as that of ES cells, which are obtained by introducing a plurality of genes (reprogramming factors) into somatic cells such as skin cells. Examples of such iPS cells include iPS cells obtained by introduction of an Oct3/4 gene, a Klf4 gene, a C-Myc gene, and a Sox2 gene and iPS cells obtained by introduction of an Oct3/4 gene, a Klf4 gene, and a Sox2 gene (Nature Biotechnology, 2008, 26, 101-106). Examples of the genes included in the reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1. These reprogramming factors may be used alone or in combination. Examples of a combination of the reprogramming factors include combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033 906, WO2010/033920, WO2010/042800, WO2010/050626, WO 2010/056831, WO2010/068955, WO2010/098419, WO2010/102267, WO 2010/111409, WO 2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat Cell Biol. 11:197-203, R.L. Judson et al., (2009), Nat. Biotech., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, Mali P, et al. (2010), Stem Cells. 28:713-720, Maekawa M, et al. (2011), Nature. 474:225-9. The iPS cells are available from certain institutes (Riken BioResource Center, Kyoto University, etc.).

Examples of the multipotent stem cells particularly include somatic stem cells such as mesenchymal stem cells, hematopoietic stem cells, neural stem cells, bone marrow stem cells, and germline stem cells. The multipotent stem cells are preferably mesenchymal stem cells, and more preferably bone marrow mesenchymal stem cells. It is to be noted that the mesenchymal stem cells broadly mean a cell group consisting of stem cells that can differentiate into all or several mesenchymal cells, such as osteoblasts, chondroblasts, and lipoblasts, and the progenitor cells thereof.

The medium according to the present invention can be suitably used for proliferation of all types of stem cells. The present medium can be used preferably for the culture of mesenchymal stem cells, ES cells, or iPS cells, more preferably for the culture of iPS cells, and particularly preferably for the culture of human iPS cells. More specific examples of the human iPS cells include a 253G1 cell line (RIKEN CELL BANK No. HPS0002), a 201B7 cell line (RIKEN CELL BANK No. HPS0063), a 409B2 cell line (RIKEN CELL BANK No. HPS0076), a 454E2 cell line (RIKEN CELL BANK No. HPS0077), a HiPS-RIKEN-1A cell line (RIKEN CELL BANK No. HPS0003), a HiPS-RIKEN-2A cell line (RIKEN CELL BANK No. HPS0009), a HiPS-RIKEN-12A cell line (RIKEN CELL BANK No. HPS0029), and a Nips-B2 cell line (RIKEN CELL BANK No. HPS0223).

### 2. Method for culturing anchorage-dependent cells

### [Culture method]

The method for culturing anchorage-dependent cells according to the present invention is characterized in that it comprises a step of culturing anchorage-dependent cells in a medium containing MFG-E8 in the absence of an anchorage. According to the medium of the present invention, it is possible to allow anchorage-dependent cells to adhere to a base material of an incubator and to allow the cells to proliferate thereon (colony formation) by action of MFG-E8, even in the absence of an anchorage. Specifically, in the present invention, MFG-E8 is able to function as an adhesion promoter for anchorage-dependent cells.

Herein, the incubator used in the culture method according to the present invention is not particularly limited. Examples of the incubator include a flask, a dish, a petri dish, a microwell plate, a microslide, a chamber slide, a tube, a tray, and a culture tank such as a culture bag or tank. The base materials of these incubators are not particularly limited, either. Examples of the base material include a glass, various types of plastics such as polypropylene or polystyrene, metals such as stainless steel, and a combination thereof. Conventionally, in order to induce anchorage-dependent cells to adhere to the surface of a base material of an incubator, the surface of the base material has been coated with an anchorage. As such an anchorage, collagen, gelatin, poly-L-lysine, poly-D-lysine, laminin, a partial structure of laminin, fibronectin, the mixtures thereof (e.g., matrigel), a cell membrane lysate preparation (Lancet, 2005, 365, p. 1636-1641), and the like are used. In the culture method according to the present invention, the coating of the surface of a base material with such an anchorage becomes unnecessary, and the present culture method can be applied to a large-scale industrial culture.

The anchorage-dependent cells may be dispersed cells upon initiation of the culture. Examples of the dispersed cells include cells that form small cell masses each consisting of several cells (typically, about 2 to 50, 2 to 20, or 2 to 10 cells).

After initiation of the culture, the anchorage-dependent cells adhere to the surface of a base material by the action of MFG-E8 and then proliferate thereon. The culture density of the anchorage-dependent cells is not particularly limited, as long as it is a density in which desired effects such as the improvement of the survival rate of the cells can be achieved. The culture density is preferably about 1.0 × 10¹ to 1.0 × 10⁷ cells/ml, more preferably about 1.0 × 10² to 1.0 × 10⁷ cells/ml, further more preferably about 1.0 × 10³ to 1.0 × 10⁷ cells/ml, and most preferably about 3.0 × 10⁴ to 1.0 × 10⁷ cells/ml.

Culture conditions such as a temperature, a CO₂ concentration, a dissolved oxygen concentration, and pH can be set, as appropriate, based on prior-art techniques. For instance, the culture temperature can be 30 to 40°C, and preferably 37°C, although it is not particularly limited thereto. The CO₂ concentration can be 1 to 10%, and preferably 2 to 5%. The oxygen partial pressure can be 1 to 10%.

### [Method for producing cell composition comprising stem cells]

In the culture method according to the present invention, stem cells are particularly used as anchorage-dependent cells, so that the present culture method can be applied to a method for producing a cell composition comprising stem cells, or to a method for producing a cell composition comprising differentiated cells from stem cells. According to the culture method of the present invention, it is possible to allow stem cells to adhere to a base material of an incubator and to allow the cells to proliferate thereon (colony formation), even in the absence of an anchorage. Therefore, by applying the culture method according to the present invention to a method for producing a cell composition comprising stem cells or differentiated cells, the coating of the surface of a base material with an anchorage becomes unnecessary, and the mixing of components derived from different animal species or immunogenic components, which are used as the anchorages, into stem cells or differentiated cells can be prevented.

The method for producing a cell composition comprising stem cells according to the present invention comprises the following specific steps, for example:
(a) a step of culturing stem cells on feeder cells,
(b) a step of dissociating the stem cells from the feeder cells, and
(c) a step of culturing the stem cells in a medium comprising MFG-E8 or a fragment of the protein in the absence of feeder cells.

Step (a) is a step of allowing stem cells to proliferate on feeder cells, while the stem cells remain in an undifferentiated state, and maintaining the cells. Step (a) may be carried out according to a conventional method for culturing stem cells and may be a culture performed in the presence of an anchorage and/or serum (or a serum extract, etc.). As feeder cells, stromal cells such as fetal fibroblasts can be used (see, for example, Manipulating the Mouse Embryo A Laboratory Manual, Second Edition, 1999, Cold Spring Harbor Laboratory Press; Gene Targeting, A Practical Approach, 1993, IRL Press at Oxford University Press; Proc. Natl. Acad. Sci. USA, 1981, 78, 7634; Nature, 1981, 292, 154; J. Virol., 1969, 4, 549; Science, 1996, 272, 722; J. Cell. Physiol., 1982, 112, 89; International Publication No. WO 01/088100; and International Publication No. WO 2005/080554).

Step (b) is a step of dissociating stem cells from feeder cells, and step (c) is a step of allowing the dissociated stem cells to adhere and allowing the cells to proliferate (form a colony) in the absence of feeder cells and an anchorage by the culture method according to the present invention. Examples of the method of dissociating stem cells from feeder cells in step (b) include a chemical method using a reagent, an enzyme, or the like, a physical method using a membrane or beads, and a combination thereof. The stem cells dissociated in step (b) may be subjected to step (a) again, so as to carry out subculture. In addition, between step (b) and step (c), a step of culturing the cells obtained in step (b) in the absence of an anchorage in a short time may also be carried out. By performing this additional step, only feeder cells are allowed to adhere to the base material of the incubator, and feeder cells mixed into stem cells can be eliminated. Moreover, in a case where serum is used in step (a), a step of washing the cells obtained in step (b) with a serum-free medium or a buffer may be carried out between step (b) and step (c).

### [Method for producing cell composition comprising differentiated cells]

In the method for producing a cell composition comprising differentiated cells from stem cells according to the present invention, differentiation of the proliferated stem cells is induced in the above-described step (c). Differentiation of stem cells can be induced according to a known method. For example, a differentiation inducer such as retinoic acid is added to a medium, so that stem cells can be differentiated into neural cells or the like in the absence of anchorage. Moreover, induction of the differentiation may also be carried out as a different step after step (c), and in such a case, the cells may be treated with a differentiation inducer in the different step in the presence or absence of MFG-E8, and preferably in the presence of MFG-E8.

Examples of the differentiation inducer that can be used include a BMP inhibitor, a Wnt inhibitor, a Nodal inhibitor, and retinoic acid. For example, in the process of inducing differentiation into chondrocytes, induction of the differentiation can be carried out by culturing mesenchymal stem cells in a differentiation induction medium (90%α MEM, 10% FBS, 2 mM L-glutamine, and 0.1 µM dexamethasone).

In addition, in the process of inducing differentiation into cardiomyocytes, 0.5 ng/ml BMP-4 is added to a medium (e.g., STEMdiff APEL Medium, STEMCELL), and one day later, the medium is replaced with a medium supplemented with 10 ng/ml BMP-4, 10 ng/ml Activin A, and 5 ng/ml bFGF. Thereafter, four days later, the medium is replaced with a medium supplemented with 10 ng/ml VEGF and 150 ng/ml Dkk1, and eight days later, the medium is replaced with a medium supplemented with 10 ng/ml VEGF, 150 ng/ml Dkk1, and 10 ng/ml bFGF, so that cardiomyocytes with autonomous beating can be confirmed.

### [Cell composition]

The cell composition according to the present invention comprising stem cells and/or differentiated cells, which have been cultured in a medium comprising MFG-E8 or a fragment of the protein in the absence of an anchorage, can be used for the further culture of stem cells, or as a cell source for regenerative medicine.

Herein, the cell composition according to the present invention can be a composition comprising dispersed stem cells like small cell masses, which is obtained by the above-described production method. The cell composition according to the present invention may comprise MFG-E8 or a fragment of the protein, which has been derived from the medium comprising MFG-E8 or a fragment of the protein used in step (c) of the above-described production method, and the present cell composition may also comprise other medium components. Further, the cell composition may also comprise feeder cells.

The cell composition according to the present invention can be used, for example, for preservation of stem cells by freeze-preservation, transportation, and subculture. When the cell composition according to the present invention is used for preservation such as freeze-preservation, the present cell composition may further comprise the aforementioned medium components, serum or a serum replacement, or an organic solvent such as DMSO, as well as a known cell preservation solution. In this case, the concentration of the serum or the serum replacement is not particularly limited and can be 1 to 50% (v/v), and preferably 5 to 20% (v/v). The concentration of the organic solvent is not particularly limited and can be 0 to 50% (v/v), and preferably 5 to 20% (v/v).

Moreover, the cell composition according to the present invention can be used as a medicament. In this case, known form and composition that are suitable for a medicament can be applied to the present cell composition. For example, a cell composition comprising cardiomyocytes can be formed into a sheet and suitably transplanted into a heart for the treatment of heart disease. The cell composition according to the present invention can be applied to patients by means such as transplantation, intravenous injection, subcutaneous injection, intra-articular injection, and intramuscular injection. The cell composition as a medicament preferably does not comprise serum, from the viewpoint of prevention of the mixing of components derived from different animal species into the cell composition.

### Examples

### [Example 1: Culture of human iPS cells in medium containing MFG-E8]

The effects of MFG-E8 in the case of culturing anchorage-dependent cells in the absence of an anchorage were evaluated using human iPS cells.

### (1) Preparation of human iPS cells

Human iPS cells (201B7, RIKEN CELL BANK No. HPS0063) were cultured on feeder cells (mouse fetal fibroblasts treated with mitomycin) (37°C, 5% CO₂). Subculture was carried out every 3 to 4 days. The subculture was carried out by dissociating the iPS cells from the feeder cells using a dissociation solution, then dispersing the iPS cells into small cell masses (about 50 to 100 masses), and seeding them on the feeder cells. The following culture medium and dissociation solution were used. Culture medium:

| | |
|---|---|
| D-MEMF12 (SIGMA) | |
| Knockout Serum Replacement (Life Technologies) | 20% |
| MEM NON-ESSENTIAL AMINO ACID [× 100] (SIGMA, Product No.: M7145) | 1% |
| L-Glutamic acid | 2.0 mM |
| 2-Mercaptoethanol | 80 µM |
| Dissociation solution: | |
| Dulbecco's phosphate buffered saline (SIGMA, Product No.: D5652) | |
| Trypsin | 0.25% |
| Collagenase IV | 1 mg/ml |
| CaCl₂ | 1 mM |

### (2) Culture in absence of anchorage

The iPS cells were dissociated as small cell masses from the feeder cells. In order to eliminate feeder cells mixed into the small cell masses, the small cell masses were transferred onto a plate coated with an anchorage (gelatin) and cultured in a maintenance culture medium (Essential-8, Life Technologies) for 1 hour, so that the feeder cells were adsorbed on the plate. It is to be noted that this maintenance culture medium was serum-free. Thereafter, the small cell masses were seeded on a plate that had not been coated with an anchorage (cell density: 1 × 10⁵ cells/0.65 cm², medium volume: 0.5 ml), and were cultured in a maintenance culture medium comprising MFG-E8 (Recombinant Human MFG-E8, R & D Systems, Catalog #2767-MF-050) (MFG-E8 2 µg/ml), or in a maintenance culture medium that did not comprise MFG-E8, for 4 days. After the culture, the area of colonies formed was measured. It is to be noted that the MFG-E8 used herein consisted of an amino acid sequence formed by adding a His tag sequence to an amino acid sequence formed by a deletion of a signal sequence in the amino acid sequence shown in SEQ ID NO: 1 (an amino acid sequence consisting of amino acids at positions 24 to 387).

### (3) Results

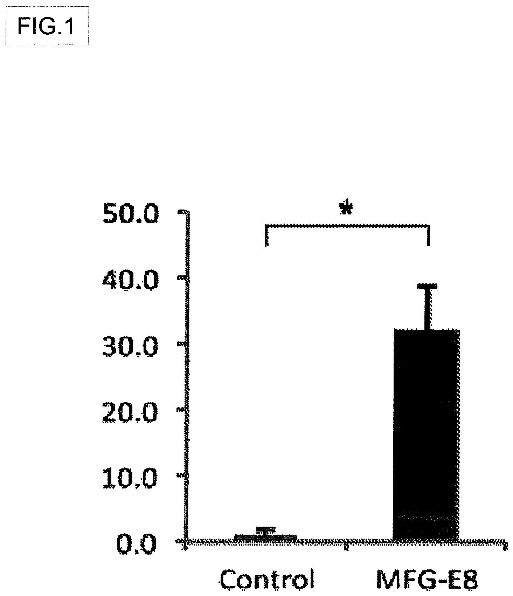

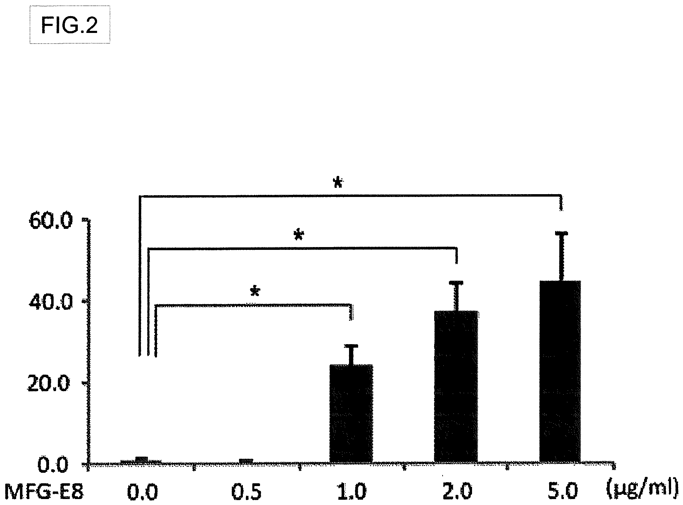

The results are shown in Figure 1. In the medium that did not comprise MFG-E8, almost no colonies were observed. On the other hand, in the medium supplemented with MFG-E8, a large number of colonies were observed. The colonies, which had grown in the MFG-E8-added medium, expressed alkaline phosphatase as a marker for undifferentiated embryonic stem cells. From these results, it became clear that MFG-E8 has the effect of promoting adhesion of iPS cells in the absence of an anchorage and enabling the survival and proliferation (colony formation) of the cells. The results obtained by carrying out an examination, while changing the concentration of the added MFG-E8 to 0, 0.5, 1, 2, and 5 µg, are shown in Figure 2. At a concentration of 1 µg/ml or more, the engraftment and growth of the cells were significantly promoted.

### [Example 2: Induction of differentiation of human iPS cells using MFG-E8-containing medium]

The differentiation ability of human iPS cells, which had been cultured using an MFG-E8-containing medium in the absence of an anchorage, was evaluated.

In the same manner as that of Example 1, small cell masses of iPS cells were seeded on a plate that had not been coated with an anchorage, and were cultured in a differentiation induction culture medium comprising MFG-E8 (MFG-E8: 1 µg/ml) or in a differentiation induction culture medium that did not comprise MFG-E8. As such a differentiation induction culture medium, a commercially available kit (PSdif-Cardio Cardiomyocyte Differentiation Kit, Stem RD) was used. It is to be noted that this differentiation induction culture medium was serum-free. After the culture for 10 days, the presence or absence of induction of the differentiation into cardiomyocytes was confirmed with a microscope.

In the medium that did not comprise MFG-E8, the engraftment of the cells was not observed, and thus, induction of the cardiomyocytes was not possible. On the other hand, in the medium supplemented with MFG-E8, cardiomyocytes exhibiting autonomous beating were found. From these results, it became clear that MFG-E8 has the effect of promoting adhesion of iPS cells in the absence of an anchorage, and enabling the survival and proliferation (colony formation) of the cells, and further, the subsequent differentiation.

### Sequence Listing Free Text

SEQ ID NO: 1: the amino acid sequence of human MFG-E8

## Claims

1. A medium for an anchorage-dependent cell, which comprises MFG-E8 (Milk fat globule-EGF factor 8) or a fragment of the protein.

2. The medium according to claim 1, wherein the anchorage-dependent cell is a stem cell.

3. The medium according to claim 2, wherein the stem cell is an embryonic stem cell, an induced pluripotent stem cell, or a mesenchymal stem cell.

4. The medium according to any one of claims 1 to 3, wherein the anchorage-dependent cell is derived from a human.

5. The medium according to any one of claims 1 to 4, which is a serum-free medium.

6. An adhesion promoter for an anchorage-dependent cell, which comprises MFG-E8 or a fragment of the protein.

7. A method for culturing an anchorage-dependent cell, which comprises a step of culturing an anchorage-dependent cell in a medium comprising MFG-E8 or a fragment of the protein in the absence of an anchorage.

8. The culture method according to claim 7, wherein the anchorage-dependent cell is a stem cell.

9. The culture method according to claim 8, wherein the stem cell is an embryonic stem cell, an induced pluripotent stem cell, or a mesenchymal stem cell.

10. The culture method according to any one of claims 7 to 9, wherein the anchorage-dependent cell is derived from a human.

11. The culture method according to any one of claims 7 to 10, wherein the medium is a serum-free medium.

12. A method for producing a cell composition comprising a stem cell, wherein the method comprises a step of culturing a stem cell in a medium comprising MFG-E8 or a fragment of the protein in the absence of an anchorage.

13. A method for producing a cell composition comprising a stem cell, wherein the method comprises:
(a) a step of culturing a stem cell on a feeder cell,
(b) a step of dissociating the stem cell from the feeder cell, and
(c) a step of culturing the stem cell in a medium comprising MFG-E8 or a fragment of the protein in the absence of a feeder cell.

14. The method according to claim 13, wherein the medium used in the step (c) is a serum-free medium.

15. The method according to any one of claims 12 to 14, wherein the stem cell is an embryonic stem cell, an induced pluripotent stem cell, or a mesenchymal stem cell.

16. The method according to any one of claims 12 to 15, wherein the stem cell is derived from a human.

17. A method for producing a cell composition comprising a differentiated cell from a stem cell, wherein the method comprises a step of inducing differentiation of a stem cell in a medium comprising MFG-E8 or a fragment of the protein in the absence of an anchorage.

18. The method according to claim 17, wherein the stem cell is an embryonic stem cell, an induced pluripotent stem cell, or a mesenchymal stem cell.

19. The method according to claim 17 or claim 18, wherein the stem cell is derived from a human.

20. The method according to any one of claims 17 to 19, wherein the medium is a serum-free medium.

21. A cell composition comprising a stem cell and/or a differentiated cell induced from the stem cell, cultured in a medium comprising MFG-E8 or a fragment of the protein in the absence of an anchorage.

22. The cell composition according to claim 21, wherein the stem cell is an embryonic stem cell, an induced pluripotent stem cell, or a mesenchymal stem cell.

23. The cell composition according to claim 21 or claim 22, wherein the stem cell is derived from a human.

24. The cell composition according to any one of claims 21 to 23, wherein the medium is a serum-free medium.

25. The cell composition according to any one of claims 21 to 24, which comprises MFG-E8 or a fragment of the protein.
